# EUROPEAN PATENT APPLICATION

(11) **EP 2 441 748 A2**
(43) Date of publication of application: **18.04.2012**
(21) Application number: 10786327.6
(22) Date of filing: 04.06.2010
(51) Int. Cl.: C07C 69/773, C08L 27/06, C08K 5/092

(54) **NOVEL PLASTICIZER FOR A POLYVINYL CHLORIDE RESIN**

(30) Priority: 09.06.2009 KR 20090050981
(71) Applicant: SK Innovation Co., Ltd., Jongno-gu Seoul 110-110 (KR); SK Global Chemical Co., Ltd., Jongro-gu Seoul 110-110 (KR)
(72) Inventor: CHUNG, Ki Nam, Daejeon 305-761 (KR); HONG, Seung Gweon, Daejeon 305-728 (KR); KWON, Tae Wook, Daejeon 305-761 (KR); WANG, Hao, Seoul 110-110 (KR)
(74) Representative: Majidi, Assieh
(86) International application number: PCT/KR2010/003609
(87) International publication number: WO 2010/143844

(57) **Abstract**

The present invention relates to an ester compound using alicyclic polyhydric acid and to a plasticizer for plastics, particularly, polyvinylchloride (PVC), including the ester compound. More particularly, the present invention relates to a plasticizer, which is derived by the esterification reaction of alicyclic polyhydric acid and alcohol. When a polyvinylchloride resin is manufactured using the plasticizer, there are advantages in that the obtained products are excellent in terms of plasticizing efficiency, and in that their physical properties, such as hardness, tensile strength and the like, can be improved.

## Description

### Technical Field

The present invention relates to an ester compound using alicyclic polyhydric acid and to a plasticizer for plastics, particularly, polyvinylchloride (PVC), including the ester compound. More particularly, the present invention relates to an asymmetric ester plasticizer, which is manufactured using alicyclic polyhydric acid, alcohol having various alkyl groups and ally alcohol containing a benzene ring, and which can be used to produce a polyvinylchloride resin composition having improved physical properties, such as hardness, tensile strength and the like, as well as a high plasticizing efficiency.

### Background Art

A polyvinylchloride resin is a homopolymer of vinyl chloride or a heteropolymer including 50% or more of vinylchloride, and is a general-purpose resin which can be used in molding methods such as extrusion molding, injection molding, calendaring or the like. Polyvinylchloride resins are widely used to manufacture various products, such as pipes, electric wires, electric appliances, toys, films, sheets, artificial leathers, tarpaulins, tapes, food wrappers, medical appliances and the like, using the molding method. Such a polyvinylchloride resin can be imparted with various processing properties by suitably adding various additives such as a plasticizer, a stabilizer, a filler, a pigment and the like.

Among the additives, a plasticizer is an essential additive which serves to impart various physical properties and functions, such as workability, flexibility, an electrical insulation properly, adhesivity and the like, to a polyvinylchloride resin by the addition thereof. In the case of a plasticizer, low volatility is a very important factor, and is important both when it is mixed in a plastic composition and when it is practically used in molded products. Further, a plasticizer must be harmless to the health so that the plasticizer can be used in the fields of foods, drinks, medicals and the like. A phthalate-based plasticizer is a typical example of such a plasticizer. However, owing to the dispute over the toxicity of regenerated plastics under laws regulating poisonous materials, it is expected that the usage of the phthalate-based plasticizer will be remarkably reduced in the future. Therefore, it is required to develop a plasticizer including an ester compound containing no phthalate as a basic backbone and having a plasticizing efficiency equal to that of the phthalate-based plasticizer.

### Disclosure

### Technical Problem

In order to solve the above-mentioned problems, the present inventors examined in depth the use of ester compounds as a plasticizer for a polyvinylchloride resin. As a result, they found that new ester compounds derived from glycerol can be used as plasticizers, and that these ester compounds are particularly excellent as plasticizers for a polyvinylchloride resin. The present invention is based on these findings.

Accordingly, an object of the present invention is to provide an asymmetric ester plasticizer, which is manufactured using alicyclic polyhydric acid having physical properties equal to or more excellent than those of a conventional phthalate-based plasticizer.

Another object of the present invention is to provide a plasticizer composition including the asymmetric ester plasticizer manufactured using the alicyclic polyhydric acid.

Still another object of the present invention is to provide a polyvinylchloride resin including the plasticizer.

### Technical Solution

In order to accomplish the above objects, a first aspect of the present invention provides a plasticizer, which is an ester compound represented by Formula 1 below: 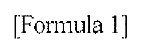 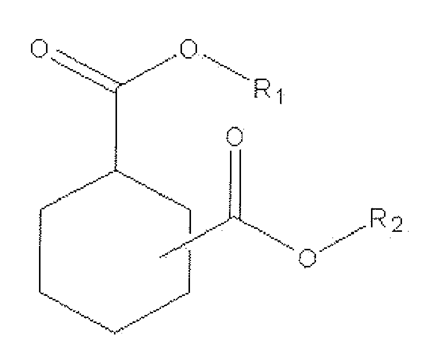 wherein R₁ is a substituted or unsubstituted ring-shaped (i.e. cyclic), branch-chain (i.e. branched) or straight-chain (i.e. liner or straight) alkyl group of 4 to 16 carbon atoms, and R₂ is a substituted or unsubstituted aryl group of 6 to 10 carbon atoms.

A second aspect of the present invention provides a plasticizer composition, comprising:
45 ∼ 99 wt% of the ester compound represented by Formula 1 above; and
1 ∼ 55 wt% of one or more compounds represented by Formulae 2 and 3: 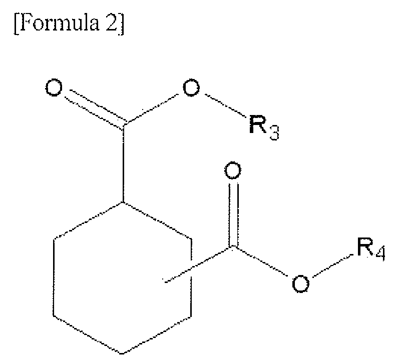 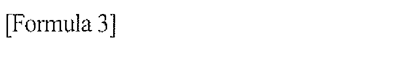 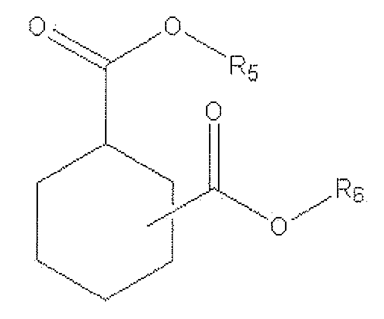 wherein R₁, R₃ and R₄ are each independently a substituted or unsubstituted ring-shaped, branch-chain or straight-chain alkyl group of 4 to 16 carbon atoms; R₂, R₅ and R₆ are each independently an aryl group of 6 to 10 carbon atoms, where the substituent groups of R₁, R₂, R₃, R₄, R₅ and R₆ are each independently selected from the group consisting of a branch-chain or straight-chain alkyl group of I to 20 carbon atoms, an alkenyl group of 2 to 20 carbon atoms, a cycloalkyl group of 3 to 20 carbon atoms, and an aryl group of 6 to 10 carbon atoms, and the substituent groups of R₁, R₂, R₃, R₄, R₅ and R₆ are the same or different.

A third aspect of the present invention provides a polyvinylchloride resin composition, comprising: 100 parts by weight of a polyvinylchloride resin; and 10 ∼ 150 parts by weight of the plasticizer composition.

### Advantageous Effects

When a polyvinylchloride resin is manufactured using the ester plasticizer using alicyclic polyhydric acid according to the present invention, there are advantages in that the obtained products are excellent in terms of plasticizing efficiency, and in that their physical properties, such as hardness, tensile strength and the like, can be improved.

### Best Mode

Hereinafter, an ester plasticizer and a polyvinylchloride resin composition including the same according to the present invention will be described in detail.

The plasticizer according to the present invention, which is an ester plasticizer obtained by the esterification reaction of diols, particularly, cyclohexane diol with various carboxylic acids, is represented by Formula 1 below: 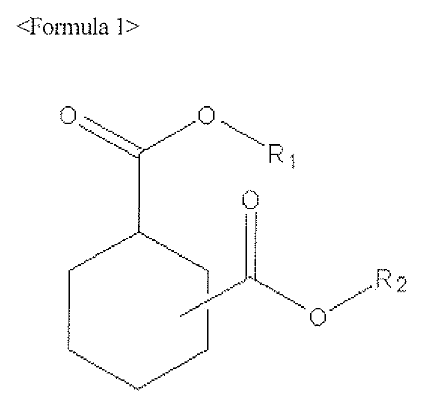 wherein R₁ is a substituted or unsubstituted ring-shaped, branch-chain or straight-chain alkyl group of 4 to 16 carbon atoms, and R₂ is a substituted or unsubstituted aryl group of 6 to 10 carbon atoms. Preferably, R₁ is a substituted or unsubstituted ring-shaped, branch-chain or straight-chain alkyl group of 6 to 10 carbon atoms, and R₂ is a substituted or unsubstituted aryl group of 6 to 8 carbon atoms. More preferably, R₁ is an octyl group or a decyl group, and R₂ is a phenyl group, a tolyl group or a xylyl group.

Further, in the substituted R₁ and R₂, a substituent group may be each independently selected from the group consisting of a branch-chain or straight-chain alkyl group of 1 to 20 carbon atoms, an alkenyl group of 2 to 20 carbon atoms, a cycloalkyl group of 3 to 20 carbon atoms and an aryl group of 6 to 10 carbon atoms, and the substituent groups of R₁ and R₂ are different or the same.

The ester plasticizer according to the present invention is an asymmetric ester plasticizer which is prepared using alicyclic polyhydric acid, an alcohol including various alkyl groups and an aryl alcohol including a benzene ring. For example, the ester plasticizer of the present invention is prepared by reacting 1,4-cyclohexane dicarboxylic acid with an acryl alcohol selectively having an alkyl group. That is, this ester plasticizer is prepared by reacting one of two dicarboxylic acids with an alcohol having an alkyl group and reacting the other thereof with an alcohol having an aryl group. The molar ratio of 1,4-cyclohexane dicarboxylic acid to alcohols is 1: 1.0 ∼ 2.0, preferably, 1: 1.2 ∼ 1.8. An acid catalyst, for example, sodium bisulfate is preferably used. As the catalyst which can be used in the present invention, there is p-toluene sulfonic acid, sulfuric acid or the like. The catalyst can be used in an amount of 0.2 ∼ 5 wt% based on a reaction mixture.

Meanwhile, examples of the solvent which can be used in the present invention include hexane, cyclohexane, toluene and xylene. It is preferred that the reaction temperature be 100 ∼ 160□. After the esterification reaction, unreacted acid and the acid catalyst are neutralized by adding an alkaline reagent, such as a sodium carbonate aqueous solution, a calcium carbonate aqueous solution or the like, thereto. The coarse ester, which was obtained by phase separation, is washed with water, dewatered and then filtered to obtain the target material.

The plasticizer of the present invention, differently from the conventional phthalate-based plasticizer, has a structure in which an asymmetric ester group is bonded to cyclohexane. This plasticizer, unlike the conventional phthalate-based plasticizer, is not harmful, and has low hardness compared to a plasticizer having a structure in which a symmetric ester group is bonded to cyclohexane, thus improving its compatibility with resin and exhibiting excellent mechanical performance.

The plasticizer composition according to the present invention includes: 45 ∼ 99 wt% of the ester plasticizer represented by Formula 1 above; and 1 ∼ 55 wt% of the compound represented by Formula 2 and/or 3. The compound represented by Formula 2 and/or 3, which is a modified compound of the plasticizer represented by Formula 1 above, exists in the plasticizer composition.

In Formula 2 or 3, R₃ and R₄ are each independently a substituted or unsubstituted ring-shaped, branch-chain or straight-chain alkyl group of 4 to 16 carbon atoms, and R₅ and R₆ are each independently an aryl group of 6 to 10 carbon atoms. Preferably, R₃ and R₄ are each independently a substituted or unsubstituted ring-shaped, branch-chain or straight-chain alkyl group of 6 to 10 carbon atoms, and R₅ and R₆ are each independently an aryl group of 5 to 8 carbon atoms. More preferably, R₃ and R₄ are each independently a hexyl group or a cyclohexyl group, and R₅ and R₆ are each independently a phenyl group, a tolyl group or a xylyl group.

Further, the substituent groups of R₃, R₄, R₅ and R₆, are each independently selected from the group consisting of a brallch-cllain or straight-chain alkyl group of 1 to 20 carbon atoms, an alkenyl group of 2 to 20 carbon atoms, a cycloalkyl group of 3 to 20 carbon atoms, and an aryl group of 6 to 10 carbon atoms, and the substituent groups of R₃, R₄, R₅ and R₆ are different or the same.

The ester plasticizer according to the present invention is generally used in a polyvinylchloride resin. This ester plasticizer may also be applied to: chlorine-containing resins, such as polyvinyl chloride, polyvinylidene chloride, polyethylene chloride, a polyvinyl chloride acetate copolymer, a polyvinyl chloride ethylene copolymer, a polyvinyl chloride propylene copolymer, a polyvinyl chloride styrene copolymer, a polyvinyl chloride isobutylene copolymer, a polyvinyl chloride-polyvinylne chloride copolymer, polyvinyl chloride-vinyl ether copolymers, and blends thereof; and synthetic resins containing no chlorine, such as an aetylonitrile-styrene copolymer, an acrylonitrilc-styrene-butadiene terpolymer, an ethylene-vinyl acetate copolymer, and blends, block copolymers and graft copolymers thereof with polyester.

The polyvinyl chloride resin composition according to the present invention includes: a polyvinylchloride resin; and the ester plasticizer composition, wherein the amount of the ester plasticizer composition to the polyvinylchloride resin is 10 ∼ 150 phr (i.e. 10 ∼ 150 parts per hundred parts). The amount of the ester plasticizer composition in the polyvinyl chloride resin composition may be suitably increased and decreased depending on the use of the polyvinyl chloride resin composition. However, when the amount of the ester plasticizer composition is less than 10 phr, flexibility or workability, which can be exhibited by a plasticizer, cannot be realized. Further, when the amount thereof is more than 150 phr, it is difficult to obtain the desired mechanical properties, and the polyvinyl chloride resin composition can be eluted, which is not preferable. Meanwhile, the method of preparing the polyvinyl chloride resin composition is not particularly limited, and the polyvinyl chloride resin composition may be prepared by any method well known to those skilled in the art.

The polyvinyl chloride resin composition including the ester plasticizer according to the present invention may be used to manufacture: building materials, such as wall-finishing materials, flooring materials, window frames, wall papers, etc.; wire coating materials; interior and exterior materials for automobiles; agricultural material such as vinyl houses, tunnels, etc.; food wrappers; film forming agents, such as sealant, plastisol, paint, ink, etc.; miscellaneous goods, such as synthetic leathers, coated fabrics, horses, pipes, sheets, toys for infants, gloves, etc.; and the like.

### Mode for Invention

Hereinafter, the present invention will be described in more detail with reference to the following Example. These Examples are set forth to illustrate the present invention more clearly, but the scope of the present invention is not limited thereto. In these Examples, physical properties were evaluated as follows.

### Hardness

Based on ASTM D2240, one point of a sample was pressed by a needle of a hardness tester (A Type) for 5 seconds, and the hardness value of the sample was measured. The hardness values of three points of each sample were measured, and then the average value of the hardness values was obtained. Hardness is used as an index for the plasticizing efficiency.

### Tensile strength, elongation, modulus at 100% elongation

The tensile strength, elongation and modulus at 100% elongation of a sample were measured using a UTM, based on ASTM D412. A dumbbell-shaped sample was pulled at a crosshead speed of 200 mm/min, and then the tensile strength, elongation and modulus at 100% elongation of the cut point of the dumbbell-shaped sample were measured. The modulus at 100% elongation thereof is the tensile strength at 100% elongation thereof, and is related closely to the plasticizing efficiency.

### Maximum torque

Maximum torque occurring at the time of mixing a polyvinylchloride resin and a plasticizer was measured using a Brabender tester.

### Example 1

### Preparation of an ester plasticizer using 1,4-cyclohexane dicarboxylic acid, benzyl alcohol and n-octanol

First, 1.0 mol of 1,4-cyciohexane dicarboxylic acid, 0.6 mol of benzyl alcohol, 0.6 mol of n-octanol, 200g of toluene (solvent), and 3.0g of sodium bisulfate (catalyst) were put into a 2L round flask provided with a stirrer and a condenser and then heated to 100°C, and then the reaction was conducted for 12 hours.

After the reaction, unreacted acid was removed by reducing the pressure to 5 mmHg at 200°C using a vacuum pump, and a reaction product was neutralized using a sodium carbonate aqueous solution (10 wt%), washed with water, dewatered and then filtered using an adsorbent to obtain an ester plasticizer composition. The obtained ester plasticizer composition is a mixture including the compound represented by Formula 1 above as a main component.

### Preparation of a polyvinylchloride resin composition

In order to evaluate the performance of the obtained ester plasticizer, a test sample was fabricated. That is, 100 parts by weight of a polyvinylchloride resin (LS-100, manufactured by LG Chem, Ltd.), 50 phr of the ester plasticizer composition including the compound represented by Formula 1 above as a main component, and 1 phr of a stabilizer (LFX-1 100, manufactured by Korea Daehyup Chem, Ltd.) were mixed, preheated to 185°C for 1 minute, pressed for 1.5 minutes and then cooled for 2 minutes to form a sheet having a thickness of 2 mm. Then, dumbbell-shaped test samples were fabricated using the sheet.

The above-mentioned test was conducted using the plasticizer and test samples, and the results thereof are shown in Table 1 below.

### Example 2

A plasticizer and a polyvinylchloride composition were prepared in the same manner as Example 1, except that para-methyl benzyl alcohol was used instead of benzyl alcohol. The test results thereof are shown in Table 1 below.

### Example 3

A plasticizer and a polyvinylchloride composition were prepared in the same manner as Example 1, except that n-decanol was used instead of n-octanol. The test results thereof are shown in Table 1 below.

### Comparative Example 1

Test examples were fabricated using di-2-ethylhexyl phthalate, which is most widely used, as a plasticizer in the same manner as Example 1. The above-mentioned test was conducted using the test samples, and the results thereof are shown in Table 1 below.

### Comparative Example 2

Test examples were fabricated using diisononyl phthalate, which is increasingly used as an alternative to di-2-ethylhexyl phthalate, as a plasticizer in the same manner as Example 1. The above-mentioned test was conducted using the test samples, and the results thereof are shown in Table 1 below.

### Comparative Example 3

Test examples were fabricated using trioctyl trimellitate as a plasticizer in the same manner as Example 1. The above-mentioned test was conducted using the test samples, and the results thereof are shown in Table 1 below.

### Comparative Example 4

A plasticizer and a polyvinylchloride composition were prepared in the same manner as Example 1, except that isonoyl alcohol was used instead of benzyl alcohol and n-octanol. The test results thereof are shown in Table 1 below.

**[Table 1]**

| Measured items | Ex.1 | Ex. 2 | Ex. 3 | Comp. Ex. 1 | Comp. Ex. 2 | Comp. Ex. 3 | Comp. Ex. 4 |
|---|---|---|---|---|---|---|---|
| Hardness, Shore A | 78 | 79 | 80 | 81 | 84 | 87 | 89 |
| Tensile strength, Kgf/cm² | 210 | 213 | 217 | 189 | 193 | 226 | 320 |
| Elongation, % | 405 | 390 | 384 | 370 | 373 | 373 | 295 |
| Modulus, Kgf/cm² | 80 | 81 | 83 | 85 | 96 | 117 | 189 |
| Maximum torque Nm | 4.5 | 4.5 | 4.3 | 4.6 | 4.5 | 4.4 | 3.9 |

From the results shown in Table 1 above, it can be seen that the plasticizing efficiency of the plasticizers of Examples 1, 2 and 3 are equal to or higher than those of the conventional plasticizers of Comparative Examples 1, 2 and 3, and that the physical properties, such as tensile strength, elongation and the like, of the plasticizers of Examples 1, 2 and 3 are equal to or higher than those of the conventional plasticizers of Comparative Examples 1,2 and 3. Further, from the results of Comparative Example 4, it can be seen that the hardness of the plasticizer in which an asymmetric ester group is bonded to cyclohexane is low compared to that of the plasticizer which has a symmetric alkyl ester group, thus improving the compatibility thereof with a resin, and that the tensile strength thereof is low and the elongation thereof is high compared to the plasticizer which has a symmetric alkyl ester group, thus improving the mechanical performance thereof. Therefore, since the novel plasticizer of the present invention has high plasticizing efficiency, it is suitable for various types of molding processes depending on various uses, and it can be utilized in various fields.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. An asymmetric ester plasticizer, represented by Formula 1 below: 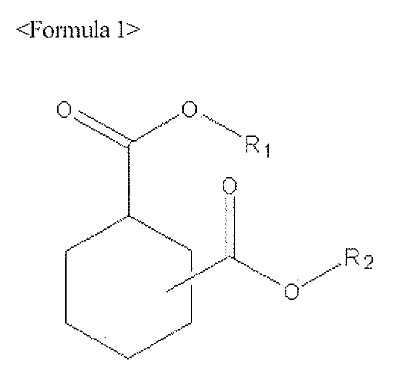 wherein R₁ is a substituted or unsubstituted ring-shaped, branch-chain or straight-chain alkyl group of 4 to 16 carbon atoms, and R₂ is a substituted or unsubstituted aryl group of 6 to 10 carbon atoms.

2. The ester plasticizer according to claim 1, wherein R₁ is a substituted or unsubstituted ring-shaped, branch-chain or straight-chain alkyl group of 6 to 10 carbon atoms, and R₂ is a substituted or unsubstituted aryl group of 5 to 8 carbon atoms.

3. The ester plasticizer according to claim 1 or 2, wherein the substituent groups of R₁ and R₂ are each independently selected from the group consisting of a branch-chain or straight-chain alkyl group of 1 to 20 carbon atoms, an alkenyl group of 2 to 20 carbon atoms, a cycloalkyl group of 3 to 20 carbon atoms and an aryl group of 6 to 10 carbon atoms, and the substituent groups of R₁ and R₂ are different or the same.

4. An ester plasticizer composition, comprising:
45 ∼ 99 wt% of a compound represented by Formula 1; and
1 ∼ 55 wt% of one or more compounds represented by Formulae 2 and 3: 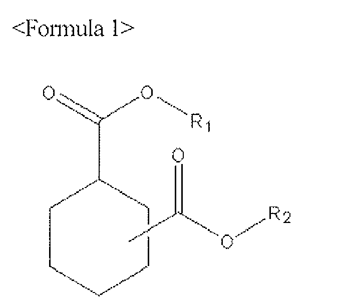 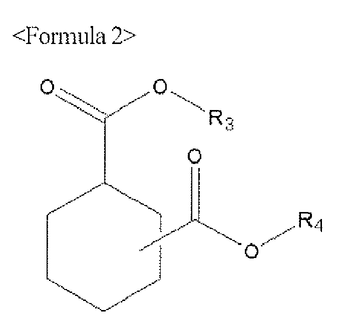 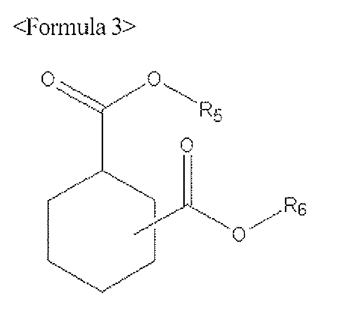 wherein R₁, R₃ and R₄ are each independently a substituted or unsubstituted ring-shaped, branch-chain or straight-chain alkyl group of 4 to 16 carbon atoms; R₂, R₅ and R₆ are each independently an aryl group of 6 to 10 carbon atoms; and the substituent groups of R₁, R₂, R₃, R₄, R₅ and R₆ are each independently selected from the group consisting of a branch-chain or straight-chain alkyl group of 1 to 20 carbon atoms, an alkenyl group of 2 to 20 carbon atoms, a cycloalkyl group of 3 to 20 carbon atoms, and an aryl group of 6 to 10 carbon atoms, and the substituent groups of R₁, R₂, R₃, R₄, R₅ and R₆ are different or the same.

5. A polyvinylchloride resin composition, comprising:
a polyvinylchloride resin; and
the ester plasticizer composition of claim 4,
wherein an amount of the ester plasticizer composition to the polyvinylchloride resin is 10 ∼ 150 phr.
